# EUROPEAN PATENT APPLICATION

(11) **EP 2 604 206 A1**
(43) Date of publication of application: **19.06.2013**
(21) Application number: 12197593.2
(22) Date of filing: 17.12.2012
(51) Int. Cl.: A61B 17/86, A61B 17/88

(54) **Bone graft fixation systems**

(30) Priority: 16.12.2011 US 201113328542
(71) Applicant: DePuy Mitek, Inc., Raynham, MA 02767 (US)
(72) Inventor: Hernandez, Joseph, Sandwich, MA Massachusetts 02563 (US)
(74) Representative: Tunstall, Christopher Stephen

(57) **Abstract**

Methods and devices are provided for securing a bone graft to a bone in a manner that ensures compression between the bone graft and bone. In one embodiment, a bone graft is positioned adjacent to a bone surface, a post is implanted in the bone, with the bone graft extending around a portion of the post, and a locking element is applied to the post to compress the bone graft into intimate contact with the bone.

## Description

### FIELD OF THE INVENTION

The present invention relates generally to methods and devices for repairing a bone defect, and in particular to methods and devices for securing a bone graft to bone.

### BACKGROUND OF THE INVENTION

The glenoid cavity is located on the upper external border of the scapula between the acromion process and the coracoid process on a bony formation known as the scapula head. The glenoid cavity is a shallow, pear-shaped, articular surface. The glenoid cavity articulates with a large, rounded head at the proximal end of the humerus, or upper armbone. The head is nearly hemispherical in form and is directed upward, inward, and a little backward. Its surface is smooth and coated with cartilage. The articular surface of the glenoid can fracture as a result of traumatic impact (avulsion) or erode over time due to repeated use or wear. The result of either of these conditions is referred to as glenoid bone loss.

Glenoid bone loss is commonly treated by replacing the damaged bone with a glenoid implant (graft). Most glenoid implants are made completely from polyethylene and affixed to the cortical bone using bone cement. Some glenoid implants have a rigid base plate made of metal, ceramic or rigid polymer with a polyethylene insert. The polyethylene material is suitable as a low friction articulating surface for engaging the humeral component. Current glenoid implants are intended to sit on a prepared surface of a glenoid bone. The surface is typically prepared by removing any remaining cartilage, reaming a smooth bony surface and by drilling receiving pockets for anchoring features or devices within the natural glenoid area. Current implant designs use either a keel or multiple elongated pegs on the back (medial surface) of the prosthetic glenoid implant as anchoring features to secure the glenoid implant inside the glenoid vault.

Glenoid implants with keeled or elongated peg anchors suffer from several disadvantages, which limit their lifespan once implanted and reduce the number of indications for which they can be used. For example, these glenoid implants can loosen due to poor fixation to the bone, and are prone to wear and fatigue failure of the polyethylene due to adhesion, abrasion, and shear stress. Because of these deficiencies, surgeons hesitate to perform glenoid replacement surgery on young or middle aged patients with glenoid articular cartilage injuries or damage due to early arthritis for fear that the implant may not last more than 10-15 years in the body, thus subjecting the patient to the possibility of two or more surgeries during the lifetime of the patient to preserve the function and pain-free state of the joint. Finally, current glenoid implants with a long keel or an elongated anchor peg are sometimes contraindicated in patients with significant glenoid bone loss. As arthritis progresses, the humeral head can wear medially and destroy the foundation of glenoid bone. In these cases, the glenoid vault can be significantly reduced in volume and depth. Thus, a typical keel or peg design can penetrate through the glenoid vault and injure the suprascapular nerve along the suprascapular notch or spinoglenoid notch with resultant denervation injury to the rotator cuff muscles. Penetrating through the bone of the glenoid vault can also fracture the body of the scapula and cause early implant loosening.

Accordingly, there is need for improved methods and devices for securing a graft to bone for use in repairing various bone defects, including defects in the glenoid.

### SUMMARY OF THE INVENTION

The present invention provides various embodiments of a bone graft fixation screw system and method of joining a bone graft to bone. In some embodiments, a method of joining a bone graft to bone includes positioning a bone graft adjacent to bone. The method can further include passing a post through a bore in the bone graft, threading a threaded distal portion of the post into the bone, and threading a washer onto a threaded proximal end of the post such that the washer advances the bone graft relative to the post and the bone to bring the bone graft into intimate contact with the bone. The method can include creating a bore through the bone graft and through the bone and can further include anchoring the post to at least one of the bone and the bone graft with a cement. Prior to placing the bone graft adjacent to bone, the bone graft can be configured to substantially conform to the shape of the bone.

In one embodiment, the post can include a thread-free intermediate portion between the threaded proximal end and the threaded distal portion. The post can be threaded into the bone using, for example, a driver tool inserted into a socket formed in a proximal end of the post. In certain exemplary embodiments, graft can be a coracoid graft and the bone can be a glenoid bone.

In another exemplary embodiment, a method for repairing a bone defect includes positioning a bone graft in contact with a surface of bone, advancing a post through a bore in the bone graft to position a distal tip of the post in contact with the surface of the bone, and rotating an inner driver coupled to the post to thread the distal tip of the post into the bone. The method further includes rotating an outer driver rotatably disposed around the inner driver to thread a washer onto a proximal end of the post with the washer compressing the bone graft toward the surface of the bone. The inner driver can be maintained in a fixed position while the outer driver is rotated.

The inner driver can have various configurations, and in one embodiment it can include a distal tip formed thereon that is disposed within a socket formed in a proximal end of the post when the post is threaded into the bone. The washer can be disposed within a socket formed in a distal end of the outer driver when the outer driver is rotated. The bone can be, for example, a glenoid bone. The method can also include forming a bore in the graft and in the bone prior to advancing the post.

In some embodiments a system for repairing a bone defect includes a post having a threaded distal portion, a threaded proximal portion, and a thread-free intermediate portion extending between the threaded proximal and distal portions. The system can further include a washer having threads formed therein and configured to threadably mate with the threaded proximal portion of the post, and a driver having an outer driver and an inner driver extending through the outer driver. The inner and outer drivers can be rotatable relative to one another, and the inner driver can be configured to engage the post and maintain the post in a fixed position while the outer driver is rotated to thread the washer onto the post. The system can further include a graft configured to be implanted in a human body in intimate contact with bone. The inner driver can include a first handle formed thereon and the outer driver can include a second handle formed thereon. A proximal end of the post can include a socket formed therein for receiving a complementary tip formed on the inner driver.

### BRIEF DESCRIPTION OF THE DRAWINGS

The invention will be more fully understood from the following detailed description taken in conjunction with the accompanying drawings, in which:

FIG. 1A is a perspective view of one embodiment of a bone implant in the form of a post for mating a bone graft to bone;

FIG. 1B is a side cross-sectional view of a washer for use with the post of FIG. 1A;

FIG. 2A is a perspective view of one embodiment of a driver tool, which can be used with the post of FIG. 1A;

FIG. 2B is an enlarged perspective view of a distal portion of the driver tool of FIG. 2A and the post and washer of FIGS. 1A and 1B;

FIG. 2C is a side cross-sectional view of the post, washer, and driver tool of FIG. 2B, shown in a mated configuration;

FIG. 3 is a side view of a bone graft positioned adjacent to a bone and having first and second holes, shown in phantom, formed therein, in accordance with one embodiment of a method of joining a bone graft to bone;

FIG. 4 is a side view of the bone and bone graft of FIG. 3, showing first and second posts implanted in the bone holes and showing the bone graft about to be advanced over the posts;

FIG. 5 is a side view of the bone and bone graft of FIG. 4, showing washers about to be attached to the posts;

FIG. 6 is a side view of a bone and bone graft having only a single post implanted therein, and showing a washer about to be attached to the post;

FIG. 7 is a side view of the bone and bone graft of FIG. 6, showing the washer advanced over a driver and about to be mated to the post; and

FIG. 8 is a side view of the bone and bone graft of FIG. 5, showing the washers attached to the posts and compressing the bone graft into intimate contact with the bone.

### DETAILED DESCRIPTION

Certain exemplary embodiments will now be described to provide an overall understanding of the principles of the structure, function, manufacture, and use of the devices and methods disclosed herein. One or more examples of these embodiments are illustrated in the accompanying drawings. Those skilled in the art will understand that the devices and methods specifically described herein and illustrated in the accompanying drawings are non-limiting exemplary embodiments and that the scope of the present invention is defined solely by the claims. The features illustrated or described in connection with one exemplary embodiment can be combined with the features of other embodiments. Such modifications and variations are intended to be included within the scope of the present invention. The terms "washer" and "graft fastener" are used interchangeably herein and can include any known fastener, except where required by context.

In general, methods and devices are provided for securing an implant, such as a bone graft, to a surface of bone. While the methods and devices can be used in a variety of surgical procedures, in an exemplary embodiment the methods and devices are used in the repair of a bony defect. For example, in one embodiment the methods and devices disclosed herein can be used in a Bristow-Latarjet procedure in which a bone graft, such as a coracoid graft, is secured to the glenoid bone of the shoulder to help prevent the humerus from slipping out of the glenoid cavity. A Bristow-Latarjet procedure can be performed arthroscopically, and is described in more detail in U.S. Publication No. 2010/0069974, published on March 18, 2010, which is hereby expressly incorporated by reference in its entirety. The various methods and devices disclosed herein allow for a safe and effective means for providing initial fixation of a bone graft to a repair site, as well as a means for maintaining compression between the bone graft and the bone throughout the healing process.

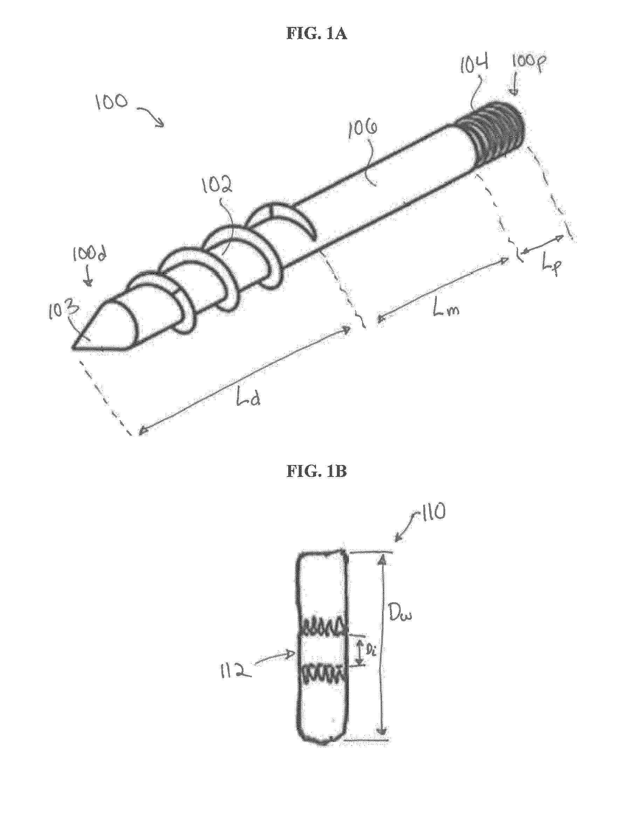

FIGS. 1A-1B illustrate one embodiment of an implant for use in attaching a bone graft, or other member, to a surface of bone. In general, the implant includes a post 100 that is configured to secure a graft to a bone. The post 100 can have various configurations, but in general the post 100 is a substantially rigid elongate member having proximal and distal ends 100p, 100d. The post 100 can vary in length depending on the intended use, but in an exemplary embodiment the length is sufficient to allow the proximal end 100p to extend proximally out of a bore in a graft when the post 100 is positioned through the graft and the distal end 100d is implanted in bone. In an exemplary embodiment, the post 100 has a length that is in the range of about 25 mm to 45 mm. The post 100 can be tapered, e.g., in a distal direction, with the proximal end 100p of the post having a diameter that is larger than a diameter at the distal end 100d of the post. Alternatively, the post 100 can have substantially the same diameter along the entire length, and only a distal-most portion or tip of the post can be tapered and can have a decreased diameter. In an exemplary embodiment the post can have a diameter that is in the range of about 3.0 mm to about 5.5 mm.

The post 100 can have various threaded and/or non-threaded regions to facilitate use of the post for attaching a graft to bone. In an exemplary embodiment, a distal portion of the post 100 is configured to fixedly attached to bone, while a mid-portion of the post is configured to freely and slidably receive a graft therearound. Such a configuration will allow the graft to be advanced along the post and into intimate contact with the bone, as will be described in more detail below. While the threaded and/or non-threaded regions can vary, in the illustrated embodiment the post 100 has a threaded distal portion 102, a threaded proximal portion 104, and a thread-free intermediate portion 106 extending between the threaded proximal and distal portions 104, 102. While the length of each threaded region and the non-threaded region can vary, the threaded distal portion 102 preferably has a length that corresponds to a length of the bone hole and/or that is sufficient to allow the post 100 to be properly secured to the bone. By way of non-limiting example, the length L_{d} of the threaded distal portion 102 can be in the range of about 40% to 60% of the total length of the post 100. For example, the length L_{d} can be in the range of about 10 mm to 27 mm depending on post length. The length of the threaded proximal portion 104 can also vary, but the length should be sufficient to allow a washer or other locking element, discussed in more detail below, to be attached to the post 100. By way of non-limiting example, the length Lₚ of the threaded proximal portion 104 of post 100 can be in the range of about 150% to 250% of the length of the washer 110. For example, the length Lₚ can be in the range of about 4.5 mm to 7.5 mm assuming that the washer is roughly 3mm in length. The length of a threaded interface can be 1.5 to 2.5 times the major diameter of the threads. The length of the thread-free intermediate portion 106 can also vary, and the length can depend on the length of the threaded proximal and distal portions 104, 102. By way of non-limiting example, the length Lₘ of the thread-free intermediate portion 106 can be in the range of about 30% to 50% of the total length of the post 100. For example, the length Lₘ can be in the range of about 7.5 mm to 12.5 mm. A person skilled in the art will appreciate that, in other embodiments, the post 100 can be threaded along the entire length, and/or that the post 100 can utilize other mating techniques instead of threads, such as flanges or other bone-engaging surface features. Moreover, as shown in FIG. 1A, the post 100 can include a distal-most tip 103 that is thread free to facilitate insertion into a bone hole. In other embodiments, the tip 103 can be self-tapping or self-drilling, e.g., such as an awl tip, drill-bit tip, or other tip designed to cut bone, and/or it can include threads formed thereon.

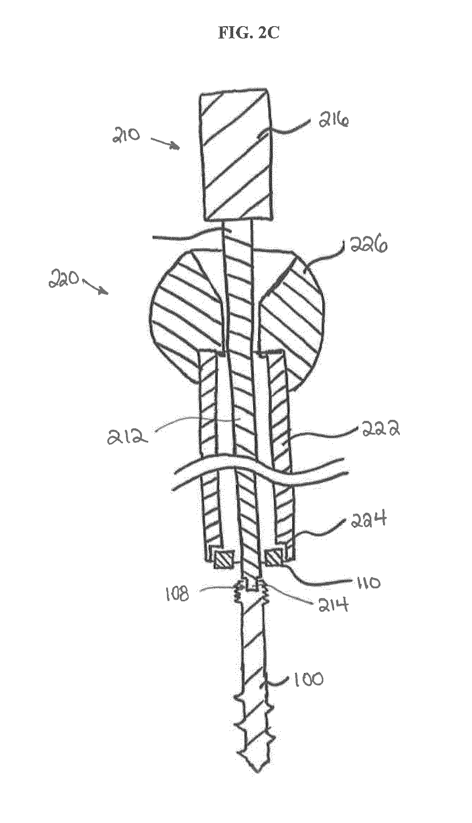

The post 100 can be formed from any biocompatible substantially rigid material such as surgical grade stainless steel, titanium, ceramics, plastics such as polyethylene, and combinations thereof. The post 100 can be solid, or it can be cannulated to allow for the passage of any desired component, such as a guidewire or surgical equipment, surgical compounds such as epoxy or cement, and debris and fluids from a patient. The post 100 can also or alternatively be configured to be coupled with another tool, such as a wrench, drill, or robotic arm. In one embodiment, the post 100 can include a drive fitting, or socket 108 (shown in FIG. 2C) for receiving a drive tool to thread the threaded distal portion 102 of the post 100 into bone and. This socket 108 can be formed in or on the proximal end 100p, at the terminal end of the proximal portion 104. In some instances the socket is formed by having a well formed in the threaded proximal portion 104 that is configured to receive a driver tool. In other embodiments the drive fitting or socket can be formed by a rigid protrusion stemming from the threaded proximal portion 104 of the post 100 and having a geometry that is designed to mate with a driver, such as a hexagonal shape (not shown).

While the intermediate portion 106 of the post 100 is preferably configured to be disposed within a bore through a bone graft such that the bone graft is freely translatable relative to or along the post 100, a person skilled in the art will appreciate that the intermediate portion 106 can include threads or other surface features, while still allowing free movement of the graft along the intermediate portion. For example, in some embodiments the graft can have a bore extending therethrough with a diameter that is greater than a maximum outer diameter of the post, thus allowing free movement of the graft along the post. In this situation, the bore formed in the bone hole preferably has a reduced diameter so as to allow the post to engage with the bone hole.

As indicated above, the threaded proximal portion 104 of the post 100 can be configured to mate with a locking mechanism, such as a washer 110. FIG. 1B illustrates one exemplary embodiment of a washer 110 that can mate to the proximal portion 104 of the post. As shown, the washer 110 has a threaded bore 112 extending therethrough and configured to threadably mate with the threaded proximal portion 104. The outer sidewall of the washer 110 can be configured to substantially conform to the shape of a driver, for example the outer sidewall can have a hexagonal geometry configured to be received by a hexagonal wrench driver. A person skilled in the art will appreciate that a variety of other locking mechanisms, such as a locking cap, can be used, and that various mating techniques can be used to mate the washer to the post. Preferably, the washer is configured to advance distally along the post to compress a graft disposed around the post toward the bone, as will be discussed in more detail below.

The washer can be formed from a variety of materials, including any biocompatible substantially rigid material such as surgical grade stainless steel, titanium, ceramics, plastics such as polyethylene, and combinations thereof. The size of the washer can also vary, but in an exemplary embodiment, the washer has a diameter D_{w} that is in the range of about 5 mm to about 8 mm, and an inner diameter Dᵢ that is configured to substantially correspond with a diameter of a post, for example the inner diameter Dᵢ can be in the range of about 2.5 mm to about 4 mm. In some embodiments, the washer 110 can be welded, epoxied, or otherwise fixed to the post after the desired compression is achieved.

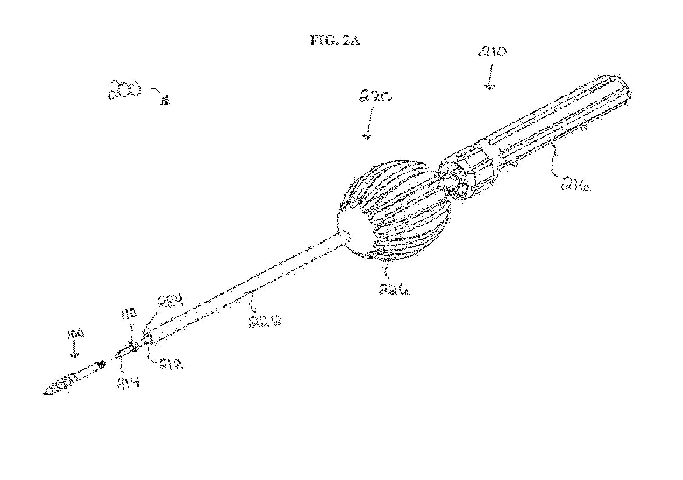

The post 100 and washer 110 can be implanted using various tools and devices known in the art, however FIGS. 2A-2B illustrate one exemplary embodiment of a driver tool 200 that can be used to implant the post 100 and washer 110. As shown, the driver 200 generally includes an inner driver 220 and an outer driver 210 disposed around the inner driver 210. The inner and outer drivers 210, 220 can be rotatable relative to one another. The inner driver 210 can be configured to engage the post 100 and to rotate and thread the post 100 into bone. The outer driver 220 can be configured to engage the washer 110 and to thread the washer 110 onto the post 100, while the inner driver 210 is maintaining the post 100 in a fixed position.

The components of the driver tool 200 can be formed from a variety of materials, and the various components can be configured to be sterilized, and/or can be configured to be disposed of after use. In an exemplary embodiment, the driver components are formed from any substantially rigid material, such as biological grade stainless steel, titanium, iron alloys, polyvinylchloride, polyethylene, and other plastics and metals, and combinations thereof. The inner driver 220 can be solid, or it can be cannulated to allow for the passage of any desired component such as a guidewire or surgical equipment, surgical compounds such as epoxy or cement, and debris and fluids from a patient. The driver 200 can also or alternatively be configured to be coupled with another tool, such as a wrench, drill, or robotic arm.

The inner driver 210 can have a variety of configurations, but as indicated above the inner driver 210 is preferably configured to engage and mate to the post 100. In the illustrated embodiment, the inner driver 210 has an elongate shaft 212 having a distal mating tip 214 and a proximal end having a handle 216 formed thereon or mated thereto. The distal mating tip 214 can have a variety of configurations, but the tip 214 preferably has a shape that complements the shape of the mating feature formed on or in the proximal end of the post 100. For example, in the illustrated embodiment the inner driver 210 includes a tip 214 having a hexagonal shape configured to be received in a hexagonal socket formed in the proximal end of the post 100. Such a configuration allows the tip 214 to extend into and engage the post such that rotation of the inner driver 210 is effective to rotate the post into bone. In other embodiments the distal tip 214 can be in the form of an Allen wrench, a Phillips head screw-driver, a flat-head screw-driver, a torque wrench, or it can have any other configuration for engaging and rotating the post 100. The handle 216 at the proximal end of the elongate shaft can also have a variety of configurations. In the illustrated embodiment, the handle 216 is in the form of a generally elongate member having flutes formed thereon to facilitate grasping of the handle 216. In other embodiments, the elongate shaft can be configured to mate to a drill or other driver for rotating the elongate shaft to drive the post 100 into bone.

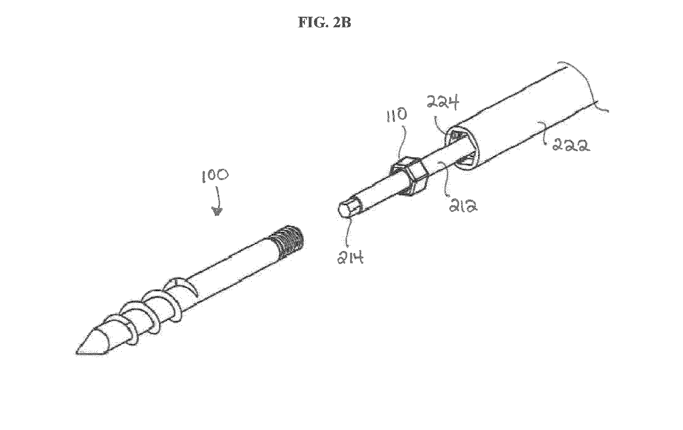

The outer driver 220 can also have a variety of configurations, but as indicated above the outer driver 220 is preferably configured to engage and mate to the washer. As shown in FIGS. 2A and 2B, the washer 110 is freely slidably disposed around the elongate shaft 212 of the inner driver 210. The outer driver 220 can include a hollow elongate shaft 222 having an opening or socket 224 formed in the distal end thereof and configured to receive and engage the washer 110. As a result, rotation of the outer driver 220 about the inner driver 210 is effective to rotate the washer 110 about the post 100. The socket 224 can have various shapes and sizes to allow the socket 224 to receive and engage the washer 110. In the illustrated embodiment, the socket 224 has a hexagonal configuration to engage the hexagonal outer surface of the washer 110. A person skilled in the art will appreciate that a variety of other techniques can be used to enable the outer driver 220 to engage and rotate the washer 110 onto the threaded proximal end of the post 100. As with the inner driver 210, the outer driver 220 can also include a handle 226 mated to or formed on the proximal end of the elongate shaft 222. The handle can have a variety of configurations, but in the illustrated embodiment the handle 226 is in the form of a bulbous member having flutes formed thereon and configured to facilitate grasping of the handle. The handle 226 can, however, have a variety of other shapes and sizes. As further shown in FIG. 2A, the handle 226 on the outer driver 220 can be positioned distal to the handle 216 on the inner driver 210. Such a configuration allows the inner driver 210 to extend through the outer driver 220. Such a configuration can also allow the inner driver handle 216 to be impacted, if necessary, to help drive the post into bone. In other embodiments, various actuation mechanisms can be used, such as a palm-grip or pistol-grip actuator. For example, the handle can be in the form of a single housing having various actuation members thereon, such as rotatable knobs, triggers, etc.

The elongate shaft on each of the inner driver 210 and the outer driver 220 can also vary, and each shaft can differ in length. In an exemplary embodiment, the shaft 212 of the inner driver 210 has a length that is greater than a length of the shaft 222 of the outer driver 220. This will allow the inner driver 210 to extend through and beyond the distal end of the outer driver 220 to allow the inner driver to engage and advance the post 100 into bone. In order to allow free slidably and rotatable movement of the inner driver 210 relative to the outer driver 220, the outer driver 220 can have an inner lumen extending therethrough (including through the handle 226) and having an inner diameter that is greater than an outer diameter of the elongate shaft 212 of the inner driver 210. The diameters and lengths can vary based on the intended use, but preferably the dimensions are configured to allow the shaft to be advanced through tissue to allow the distal end to be positioned adjacent to bone, with the handles 216, 226 positioned outside of the patient's body.

FIG. 2C illustrates the driver tool 200 in use, showing the distal tip 214 of the inner driver 210 extending into the socket 108 formed in the post 100, and showing the washer 110 disposed within the socket 224 formed in the distal end of the outer driver 220. As shown, rotation of the inner driver 210 will be effective to rotate the post, independent of the outer driver 220 and the washer 110, thus allowing the post 100 to be driver into bone. Once implanted, the inner driver 210 can be held in a fixed position, and the outer driver 220 can be rotated about the inner driver 210 to rotate and thread the washer 110 onto the proximal threaded end of the post 100. As will be discussed in more detail below, advancement of the washer 110 along the post 100 in a distal direction will advance a bone graft slidably disposed around the thread-free intermediate portion in a distal direction, thereby placing the bone graft into intimate contact with the bone having the distal end of the post implanted therein.

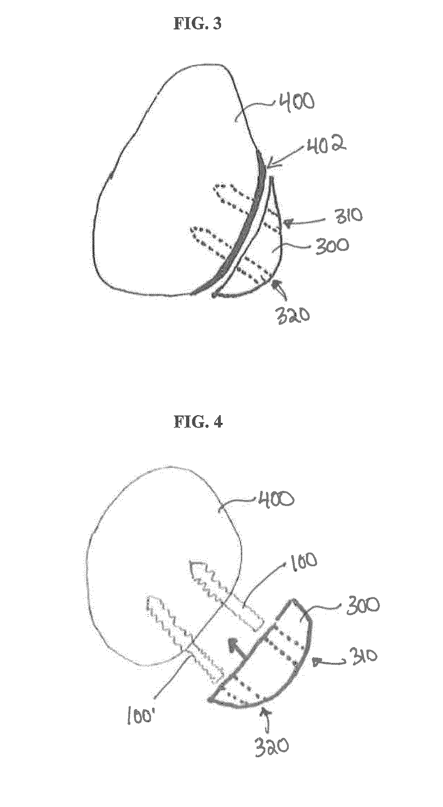

FIGS. 3-8 illustrate various exemplary methods for implanting a bone graft, and in particular for attaching a bone graft to a surface of bone, with the bone graft being compressed or held in intimate contact with the surface of the bone. While the methods are discussed in connection with post 100, washer 110, and driver tool 200, a person skilled in the art will appreciate that the methods can be performed using any implant or tool that is configured to compress the bone graft into the bone surface.

FIG. 3 illustrates a bone graft 300 positioned adjacent to a surface of bone 400. The bone graft 300 can have any shape and size, and can be formed from a variety of materials, including metals, plastics, or other synthetic materials, as well as autograft and allograft bone, and combinations thereof. Exemplary bone grafts include, for example, a portion of the coracoid process ("coracoid graft"). In some embodiments, the graft 102 can be made completely from polyethylene. In other embodiments, the graft 102 can have a rigid base plate made of metal, ceramic or rigid polymer with a polyethylene insert.

The bone 300 can be, for example, a glenoid bone or alternatively any type of bone requiring a graft. In various embodiments, the graft 300 can be shaped to substantially conform to the geometry of the bone 400 at the graft fixation site 402 (also referred to herein as the repair site). The graft fixation site 402 is used herein to refer to the surface of the bone where contact between the bone graft 300 and bone 400 will occur. This region can be predetermined, allowing for the forming of the graft 300 to substantially conform to the predetermined geometry of the graft fixation site 402.

In preparation for bone graft fixation, one or more holes or bores can be formed through the graft 300 and the bone 400. In the illustrated embodiment, two bores 310, 320 are formed through the graft 300 and the bone 400. The bores 310, 320 can be drilled with any known tool, and they can be produced simultaneously, such as by drilling the bores 310, 320 through both the graft 300 and bone 400 after the graft 300 is placed adjacent to the bone 400. Alternatively, the graft 300 can have pre-drilled bores and the bores in the bone 400 can be formed independently, either using the graft 300 as a template or separately without use of the graft. The bores in the bone 400 can optionally be tapped to substantially correspond to the threaded portion of a post 100.

As shown in FIG. 4, a post 100, 100' is positioned in each bore 310, 320 in the bone 400. In some embodiments, after placing the graft 300 adjacent to the bone 400, with the bores 310, 320 substantially aligned, each post 100, 100' can be passed through the graft 300 into the bone 400. In an alternate embodiment, as shown, the post 100, 100' can be implanted directly into the bone 400, with the graft removed. The graft 300 can then be advanced over the proximal ends of the posts 100, 100' to position the graft 300 along the thread-free intermediate portion. While not shown, the driver tool 200 can be used to implant each post. For example, the inner driver can be passed through the graft 300 and the distal tip can be used to drive the post into bone.

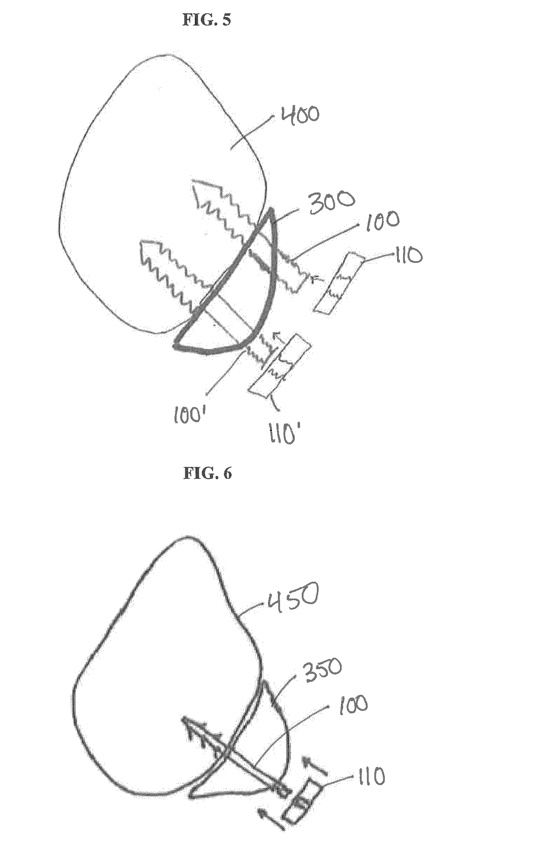

Once the posts are implanted and the graft 300 is positioned around the posts, the washers can be mated to the proximal threaded region of each post 100, 100'. FIG. 5 illustrates the graft 300 positioned adjacent to the bone 400, and first and second washers 110, 110' about to be mated to the proximal threaded region of each post 100, 100'. Where the driver tool 200 is used, the inner driver can remained extended through the graft and in engagement with one of the posts, e.g., post 100, and the outer driver have the washer, e.g., washer 110, disposed in the distal end thereof. The outer driver can then be advanced distally along the inner driver to position the washer 110 on the proximal end of the post 100. The outer driver can then be rotated relative to the inner driver to thereby rotate the washer relative to the post, thus threading the washer onto the proximal end of the post. As the washer is threaded, the washer 110 will abut the graft and eventually will apply a force to the graft 300 to push or compress the graft toward the bone 400. Since the post 100 is implanted in the bone 400, the post and bone will remain fixed, as the bone graft 300 is compressed between the bone 400 and the washer 110. The free sliding movement of the graft 300 relative to the post 100 will allow for such compression. As a result, the graft 300 is advanced into intimate contact with the bone 400, and is thereby securely fixed to the bone 400. The washer 110 can be threaded to the extent necessary to achieve the desired compression. This compression force allows the graft 300 to be secured without placing undue stress on either the graft 300, the bone 400, or the bore(s), thus allowing a strong fixation of the graft 300 to the bone 400 that is resistant to loosening, wear, or fatigue.

In some embodiments, the post can be further anchored in the bone 400 with a cement, wherein the cement can be any adhesive material, such as polymethylacrylate. Care can be taken to ensure that any bore in the bone does not penetrate through the bone of the glenoid vault, damage the scapula or suprascapular nerve, or otherwise damage the bone or its surroundings. The cement can be passed through any lumen in the driver tool, for example cement or other bone-growth promoting materials can be passed through the inner driver, and through the post. The post can include openings formed in the sidewalls and/or distal end thereof to allow the materials to seep out and fill any space between the post and the bone. A person skilled in the art will appreciate that a variety of other techniques can be used to introduce bone-growth promoting and/or affixation materials into the bone hole.

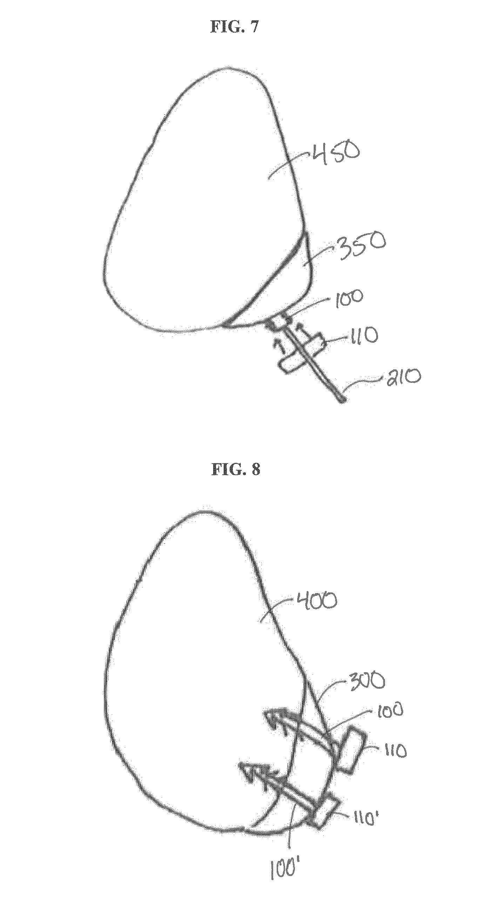

A person skilled in the art will appreciate that the graft can be secured to bone using any number of posts. For example, FIG. 6 illustrates a single post 100 inserted through a graft 350 and implanted in bone 450. A washer 110 can be applied to the post 100 to compress the graft 350 between the washer 110 and the bone 450. As is shown in FIG. 7, when tightening the washer 110 along a threaded proximal portion of the post 100, the inner driver 210 is used to immobilize the post 100. By immobilizing the post 100 during washer 110 actuation, the post 100 is not able to rotate, thus insuring that the post 100 does not rotate beyond a desired position so as to avoid stripping a tapped bore through the bone 450. An outer driver (not shown) that is rotatably connected to the inner driver 210 can be used to mate with and actuate the washer 110 without actuating the post 100, but any known driver or tool, such as but not limited to an Allen wrench, a torque wrench, a Phillips head screw-driver, or a flat-head screw driver, can be used to immobilize the post while threading the washer onto said post.

While not shown, in other embodiments, a plurality of washers can be employed. In such embodiments, the washers can be positioned to allow for either a uniform compression along the graft fixation site, or alternatively the washers can be compressed such that the compression force applied to each of a plurality of posts varies along the graft fixation site.

FIG. 8 depicts bone graft 300 fixed to the surface of a glenoid cavity 400 by a compression force acting along the plane of the posts 100, 100'. The compression force is provided by washers 110, 110'. The washers are disposed along an outer surface of the bone graft and are positioned to provide the optimum amount of compression along the graft fixation site. In some embodiments, the washers can be counter-sunk within the graft so as to yield a surface that is substantially smooth or rounded (not shown). When the desired compression is achieved, the proximal end of the post can terminate in a manner so as to remain flush with the washers, or can terminate either within the washers or can extend outside of the washers (not shown).

In some embodiments, the bone graft fixation system can be compiled in a sterile kit for joining a bone graft to bone. The kit can comprise a post, a driver tool, and a graft. The kit can further be configured to join a bone graft to a glenoidbone in a Bristow-Laterjet type procedure as described herein. The kit can further include various tools, devices, and materials for performing arthroscopic surgery, such as sutures, scalpals, forceps, and optical equipment. Furthermore, some or all of the kit can be disposable and sterilized.

One skilled in the art will appreciate further features and advantages of the invention based on the above-described embodiments. Accordingly, the invention is not to be limited by what has been particularly shown and described, except as indicated by the appended claims. All publications and references cited herein are expressly incorporated herein by reference in their entirety.

## Claims

1. A system for repairing a bone defect, comprising:
a post having a threaded distal portion, a threaded proximal portion, and a thread-free intermediate portion extending between the threaded proximal and distal portions;
a washer having threads formed therein and configured to threadably mate with the threaded proximal portion of the post;
a driver having an outer driver and an inner driver extending through the outer driver, the inner and outer drivers being rotatable relative to one another, and the inner driver being configured to engage the post and maintain the post in a fixed position while the outer driver is rotated to thread the washer onto the post.

2. The system of claim 1, further comprising a graft configured to be implanted in a human body in intimate contact with bone.

3. The system of claim 1 or claim 2, wherein the inner driver includes a first handle formed thereon and the outer driver includes a second handle formed thereon.

4. The system of any preceding claim, wherein a proximal end of the post includes a socket formed therein for receiving a complementary tip formed on the inner driver.
